(19) **Europäisches Patentamt / European Patent Office / Office européen des brevets**

(11) **EP 4 616 841 A2**

(12) # EUROPEAN PATENT APPLICATION

(43) Date of publication:
**17.09.2025 Bulletin 2025/38**

(21) Application number: **25187273.5**

(22) Date of filing: **20.06.2023**

(51) International Patent Classification (IPC):
**A61K 8/73** (2006.01)

(52) Cooperative Patent Classification (CPC):
**A61Q 5/06; A61K 8/042; A61K 8/73; A61K 8/733; A61Q 19/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.06.2022 EP 22180873**

(62) Document number(s) of the earlier application(s) in accordance with Art. 76 EPC:
**23730836.6 / 4 429 776**

(71) Applicant: **BASF SE**
**67056 Ludwigshafen am Rhein (DE)**

(72) Inventors:
• **Erasmy, Jessica**
  **40589 Düsseldorf (DE)**
• **Grund, Patricia**
  **40589 Düsseldorf (DE)**
• **Bhattacharya, Sandip**
  **40589 Düsseldorf (DE)**
• **Schäfer, Dörte**
  **40589 Düsseldorf (DE)**
• **Falkowski, Jürgen**
  **40589 Düsseldorf (DE)**

(74) Representative: **BASF IP Association**
**BASF SE**
**GBI - Z078**
**67056 Ludwigshafen (DE)**

Remarks:
This application was filed on 03-07-2025 as a divisional application to the application mentioned under INID code 62.

(54) ## A COMPOSITION COMPRISING POLYMERS

(57) The present invention relates to a composition suitable for cosmetic applications, especially suitable for styling hair or for skin care purposes, comprising at least two of the components konjac gum, tara gum and algin, wherein the weight ratios of these components are within a specified range. Furthermore, the present invention relates to a raw material composition consisting of at least two of the said components, wherein this raw material composition can be used to make the composition according to the present invention. Furthermore, the present invention relates to the use of the composition for styling hair or for skin care.

EP 4 616 841 A2

**EP 4 616 841 A2**

**Description**

The present invention relates to a composition suitable for cosmetic applications, especially suitable for styling hair or for skin care purposes, comprising at least two of the components konjac gum, tara gum and algin, wherein the weight ratios of these components are within a specified range. Furthermore, the present invention relates to a raw material composition consisting of at least two of the said components, wherein this raw material composition can be used to make the composition according to the present invention. Furthermore, the present invention relates to the use of the composition for styling hair or for skin care.

Konjac gum and glucomannan are used synonymously in the present document, both are abbreviated by either Glu or K. Algin is abbreviated by Alg or A and tara gum is abbreviated by Tar or T.

Sustainability and natural aspects are of increasing importance in the cosmetic industry. Discussions around microplastic are influencing this strongly. Therefore, the need to provide products which are sustainable is a major market need. This leads to a challenge to cosmetic formulations, in which polymers play a major role for either thickening and stability or texture of the formulations.

Synthetic polymers are widely used in cosmetic formulations for these aspects. The texture of synthetic polymers is well known and liked by consumers. Natural replacements for these synthetic polymers can be natural biopolymers gained from plants. These polymers have advantages in naturality but come along with weaknesses in formulation performance. One of the major weaknesses seen by consumers is the texture of the products, which is often either slimly or watery.

There is a need to overcome the challenges in formulating with biopolymers. There is a need to achieve an attractive, soft, and nice texture without scarifying other aimed at performance parameters.

The online-publication on the platform IP.com having the IP.com number IPCOM000268678D discloses hair styling mousses with natural polymers. Formulations comprising xanthan gum and algin are disclosed.

The problem underlying the present invention is to provide a composition suitable for a cosmetic application, wherein this composition shall have an acceptable texture (texture as defined in more detail in the present document), i. e. a texture that is perceived as better than the texture provided by xanthan gum. Furthermore, preferably, when the composition shall be used as a hair styling formulation, the composition shall have good hair styling properties, i. e. shall provide high bending stiffness, high curl retention and low flaking.

This problem is solved by the composition according to claim 1 of the present document. This composition is a subject of the present invention.

A further subject of the present invention is the raw material composition according to claim 2 of the present document. This raw material composition consists of two or three components as defined in the claims of the present document. In this context consisting of means that no other substances are present in substantial amounts. The presence of impurities or residual humidity, however, is not excluded by the words consisting of.

Further subjects of the present invention are the use according to claim 7 and according to claim 8 of the present document.

Further subjects of the present invention are the processes according to claim 9 and according to claim 10 of the present document.

The subject-matters of the dependent claims are specific embodiments of the present invention.

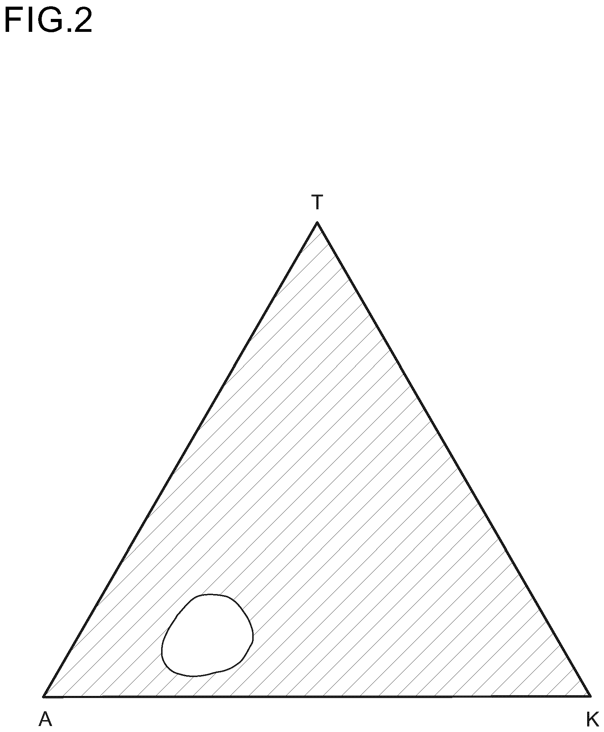

The claims of the present document refer to drawings, namely Fig. 2 to 4, of the present document for defining the subject-matter claimed. It is possible, however less accurate, to define the subject-matter claimed without referring to drawings. This can be done by approximating the hatched surfaces of the drawings with polygons. This is described in detail in the section describing the drawings of the present document.

Suitable further cosmetically acceptable ingredients can be any cosmetically acceptable ingredient. These ingredients are known to the person skilled in the art and can be found in several publications, e. g. in the latest edition of the "International Cosmetic Ingredient Dictionary and Handbook" published by the Personal Care Products Council. Another well-known source of further suitable cosmetically acceptable ingredients is the cometic ingredient database CosIng. CosIng can be accessed on the internet pages of the European Commission.

In the following paragraphs the term "polymer mixture" is used as a synonym for the mixture of components, e. g. konjac gum plus tara gum plus algin, in the composition according to the present invention.

The composition according to the present invention preferably comprises the polymer mixture in a total polymer content of 0.2-5 % by weight.

Suitable further cosmetically acceptable ingredients can be selected from the group consisting of preservatives, humectants, neutralizing agents, calcium salts, emollients, emulsifiers, fragrances and actives, plasticizers, glycerin, sodium benzoate, citric acid, calcium chloride, conditioning agents and combinations thereof.

Suitable further cosmetically acceptable ingredients can be selected from the group consisting of glycerin, sodium benzoate, citric acid, calcium chloride and combinations thereof.

The polymer mixtures according to the present invention can be comprised in the composition according to the present invention, which can be a gel, a cream gel or any textured cosmetic formulation, typically at a concentration of 0.2 to 5 % by weight. The composition according to the present invention can further comprise a neutralizing agent, typically in an amount that results in a pH of the composition (e. g. a gel) of pH = 4.0 to 7.5. The neutralizing agent can be a carboxylic acid, e.g. lactic acid or another alpha hydroxy acid such as malic, citric, and tartaric acid. The composition according to the present invention can further comprise a humectant e.g. panthenol, glycerol or propylene glycol typically at a concentration of 0.0 to 10.0 % by weight. The composition according to the present invention can comprise a polymer chosen from cationically charged polymers or cationic surfactants e.g. Guar Hydroxypropyltrimmoniumchloride, Cetrimonium Chloride, typically at a concentration of 0.01 to 2.0 % by weight. The composition according to the present invention can further comprise a solubilizing agent e.g. PEG-40 Hydrogenated castor oil or Lauryl Glucoside typically in a concentration of 0.2 to 1.0 % by weight. The composition according to the present invention can further comprise a preserving agent, e.g. phenoxyethanol, ethylhexylglycerine and benzoic acid, typically in a concentration of 0.1 to 1.0 % by weight. The composition according to the present invention can comprise emollients typically at a concentration of 0.2-10 % by weight. The composition according to the present invention can also comprise waxes and other consistency agents typically in a concentration of 0.1-5 % by weight, or further emulsifiers typically in a concentration of 0.05 % to 5.0 % by weight. The composition according to the present invention can also comprise actives, eg. proteins and their derivatives and plant extracts, typically in a concentration of 0.1- 2 % by weight.

The composition according to the present invention can have the following composition in % by weight (abbreviations as explained in the experimental section): 1.35 % Glu and 0.15 % Alg - or 1.00 % Glu and 1.00 % Alg - or 0.66 % Glu and 0.66 % Alg and 0.66 % Tar - or 1.68 % Glu and 0.25 % Alg and 0.07 % Tar - or 1.25 % Glu and 0.65 % Alg and 0.10 % Tar - or 0.60 % Glu and 0.78 % Alg and 0.62 % Tar - or 0.75% Glu and 0.75 % Alg, and in each case 3.00 % glycerin and 0.01 % calcium chloride and 0.50 % sodium benzoate and 0.09 % citric acid and 0.30 % perfume, the balance being water.

**Examples**

In this experimental section and in all other parts of the present document % means % by weight, unless specified differently.

A ternary system consisting of a combination of three different biobased polymers, has been examined to determine the ranges within the ternary system having advantageous properties with respect to cosmetic applications, especially with respect to hair styling applications.

The following ternary systems has been examined:

- konjac gum, tara gum and algin

The following commercially available products, obtainable from BASF SE, Germany, have been used and the following abbreviations are used in the present document:

|  |  |  |
|---|---|---|
| • konjac gum | (Glu) | Verdessence® Glucomannan |
| • tara gum | (Tar) | Verdessence® Tara |
| • algin | (Alg) | Verdessence® Alginate |

The following properties of these ternary systems have been examined:

- texture
- bending stiffness
- flaking (including number of flakes, particle size and area fraction)
- curl retention

This examination of four properties resulted in six parameters that have been determined, three parameters characterizing flaking (number of flakes, particle size and area fraction), one parameter for texture, one parameter for bending stiffness and one for curl retention.

For examining the properties of the ternary system a design of experiment (DoE) approach has been used which is described in more detail in the following paragraphs.

To perform the experiments for the mixtures of the three polymers A, B and C Design of Experiments (DoE) was used. DoE was performed by Minitab® 20.3, a software available from Minitab Inc. To create the map of experiments a simplex matrix for two to ten components was chosen. The measurement-points in a simplex matrix are evenly distributed in a triangular shaped diagram including one measurement-point with equal amount of each polymer located in the center of the diagram. In the present case there were three components, which were three polymers of a ternary polymer-mixture.

The map of experiments arranged in a triangular diagram contained 19 measurement points with different amounts for each polymer in each mixture. Minitab® 20.3 required input at these 19 measurement points. These 19 measurement points were located at the following positions within the triangular diagram:

- three measurement points were at the corner of the triangular diagram (representing a pure polymer)
- nine measurement points were located on the lines of the triangular diagram, thus representing a mixture of two polymers
- one measurement point was in the center of the triangular diagram, representing a mixture of three polymers, wherein the amount of all three polymers was the same
- six measurement points were arranged symmetrically around the center-point.

The plan of experiments was given to the experimenter in a random way without any repetitions.

After the experiments were done (i. e. after the four properties to be examined had been determined at each of the 19 measurement points) and the corresponding results were available Minitab® 20.3 was used again to fit the experimental data into the best mathematical model available for each of the six parameters. For each parameter the mathematical model of the following list was chosen that described the results in the best way.

- Linear model: *property* = $c_1 * A + c_2 * B + c_3 * C$
- Quadratic model: *property* = $c_4(A * B) + c_5(A * C) + c_6(B * C)$
- Specific cubic model: *property* = $c_7(A * B * C)$
- Fully cubic model: *propery* = $c_8[A * B(A - B)] + c_9[A * C(A - C)] + c_{10}[B * C(B - C)]$
- Specific quartic model: *property* = $c_{11}(A * A * B * C) + c_{12}(A * B * B * C) + c_{13}(A * B * C * C)$
- Fully quartic model: *property* = $c_{14}(A * B * (A - B)^2) + c_{15}(A * C * (A - C)^2) + c_{16}(B * C * (B - C)^2)$

($c_1$, $c_2$, ... $c_{16}$ = coefficients are coming from data-modelling; if a coefficient is equal to zero this term does not apply)

The person using Minitab® 20.3 could also chose a combination out of the different models. To choose the best mathematical model for each property for each ternary system the p-value for each model was not allowed to be above 0.05. Additionally, the $R^2$-value had to be above 65 %.

The meaning of p-value is well-known in the art. The p-value is used to decide if a certain pattern of measured results is statistically significant. If the p-value is 0.05 or lower, the result is stated as significant. If the p-value is above 0.05 the model is statistical not significant and therefore it would result in overfitting. In this case the coefficients ($c_1$, $c_2$, ... $c_{16}$) are set to zero for the particular model.

The meaning of $R^2$-value is well-known in the art. The $R^2$-value is a statistical measure of fit that indicates how much variation of a dependent variable is explained by the independent variable(s) in a regression model. If $R^2$ is equal to 100 % the experimental data and the mathematical model are exactly the same which is never the case. From our experience a value of 65 % and higher gives a reliable model.

If that was not the case (p-value not above 0.05 and $R^2$-value above 65 %) two or three models were compared to each other and the decision for the better model was made by comparison of the PRESS- and $R^2$(prediction)-value.

PRESS, a parameter well-known in the art, means "Predicted Residual Sum of Squares". The fit of a model is proved by using a sample of observations that were not used to estimate the model. The lower the value the better is the predictive ability of the mathematical model.

$R^2$ (prediction) is a parameter well-known in the art. To calculate $R^2$ (prediction) each measurement-point is systematically removed from the data set, estimating the regression equation, and determining how well the model predicts the removed measurement-point. If the model is overfitted $R^2$ (prediction) is lower.

By carrying out the described DoE experiments and modelling it could be determined in what areas of a ternary plot (diagram), representing the composition of the ternary polymer-systems examined, the six properties that have been examined, are above (or below) a chosen threshold-value.

To find the optimal areas which is equivalent to the best mixtures in the diagram a minimum requirement for each property was set. This minimum requirement values were set in consideration of standard hair-styling polymers available on the market.

For the property texture, the target was to be more acceptable in perception than the current biopolymer standard on the market for hair gels, which is currently xanthan gum.

For the property bending stiffness and curl retention the min max values were set in consideration of synthetic polymers on the market, like PVP, VP/VA copolymer and VP/Methacrylamide/Vinyl Imidazole Copolymer, which provide different bending stiffnesses. The target was to be as close as possible to this synthetic standard or being even better, which means higher where possible.

For the property flaking the min max values were set in consideration of synthetic polymers on the market, like PVP, VP/VA copolymer and VP/Methacrylamide/Vinyl Imidazole Copolymer, which provide different flaking values. The target was to be as close as possible to this synthetic standard or being even better, which means lower where possible.

The optimal areas in the ternary plots (diagrams), which means the optimal mixtures for hairstyling/gel formulations are defined by the overlap of the minimal requirements in these diagrams of all four properties.

In the following paragraphs the methods for determining texture, bending stiffness, flaking and curl retention are described in detail.

### Texture

A sensory assessment was performed to assess the soft and creamy texture of the samples to be evaluated. The evaluation was done by three trained volunteers.

The results were calculated as a median from the three evaluations).

The target was to be different to the watery and simely texture of common natural solutions, which are state of the art but not very much liked by the consumers.

Textures were evaluated in a grading from 1 to 4 in 0.5 steps (grade 1 was the highest grade while 4 was the lowest one).

Standard texture of Xanthan gum was set to 3 as a reference. Standard texture of Alginate was set to 1.5 as a reference.

### Bending Stiffness Test

Caucasian, dark brown hair strands of 1.8 g weight, 2 cm width and 8 cm length of free hair, flat shaped and with a gluing length of 2 cm x 2 cm soft swatch were used for the test. Seven clean and dry hair strands were used for each sample.

For the purposes of testing the hair gels were diluted with deionized water in a ratio 1:4. The applied amount was 1.4 g per strand. The hair strands were combed with the fine side of the comb until the strands were homogeneous. The scattered parts were brought together between two fingers without exerting pressure. The hair strands were treated in a special channel to create a flat and uniform shape.

The formed hair strands were then hung vertically in a rack, where the hair strands were left to dry for at least 1 hour under normal laboratory conditions. After drying the rack and strands were placed in a climate chamber at 65 % relative humidity and 21 °C overnight.

The maximum force needed to break the hair strands was then measured in cN using a device such as a texture analyzer with a 3 point bending stiffness test set-up, which can measure force required until breaking point e.g. a Diastron. The treated hair strand was bent to breaking point by the probe which was connected to a force measurement cell. The measurement was repeated for 7 hair strands in order to obtain a mean value of maximum force before breakage. The bending stiffness value is given as maximum force in cN.

### Flaking

After measuring the bending stiffness, the dry hair strands were combed using a material testing machine (supplier: Zwick / Z005) to generate flaking on the hair strands. Images of the hair strands were taken in a photo box, where a circular light and a polarizing filter were used to control the light conditions and avoid reflections. The pictures were evaluated by a software package (open platform: ImageJ) to determine the number of flakes on the hair strands and the mean particle size of the flakes. Area fraction was calculated from the number of flakes, the mean particle size of the flakes and the surface area of the hair strand.

Intensity of flaking was determined using the following three parameters: number of flakes, particle size of flakes and the area fraction, which describes the area covered by particles. All three parameters indicate a lower flaking the lower their value is.

### Curl Retention

Dark brown hair strands of 2 g free hair, 15 cm length, round shaped were used. The hair strands were bleached with 5 % hydrogen peroxide before use. The gel was diluted with deionized water in ratio 1:2. The applied amount was 1.5 g per strand. The 5 hair strands were combed with the fine side of the comb until the strands were homogenous. The scattered parts are brought together between two fingers without exerting pressure.

The hair strands were wound onto Teflon curlers with guides to ensure consistent curling over all strands. The curlers with hair strands were then dried at 40°C overnight and were subsequently cooled for 1 hour. Being hung on a rack with a scale into a climate chamber at 25 °C and 90 % rh the initial length of the strand and the length after 5 h and 24 h was recorded accordingly.

The curl retention (CR) was then calculated using the following formula

$$CR \text{ in } \% = [(L-Lt)/(L-L0)] \times 100$$

Where:

L = Length of hair (15cm)
L0 = Length of hair curl, start
Lt = Length of hair curl, after a certain period of time (5h/24h)

The examination of texture, bending stiffness, flaking and curl retention of the ternary systems has been done using formulations comprising the ternary systems. Initial tests have been performed to determine the total polymer content in a styling formulation which resulted in a useful formulation. This has been done based on concentration rows from 0.5 % to 5 %. Concentrations of synthetic polymer in formulations commonly used have been considered in this set up. Surprisingly it has been found that half of the concentration commonly used in the case of synthetic polymers led to the aspired texture and performance.

The following formulations have been used for the DoE measurements described.

**Glu, Tar, Alg (total polymer content 2 %):**

| Mixture no. | 203 | 204 | 205 | 206 | 207 |
|---|---|---|---|---|---|
| Ingredients | Mass Fraction [%] | Mass Fraction [%] | Mass Fraction [%] | Mass Fraction [%] | Mass Fraction [%] |
| WATER DEMIN | 94.35 | 94.31 | 94.08 | 93.68 | 94.04 |
| SODIUM BENZOATE | 0.50 | 0.50 | 0.50 | 0.50 | 0.50 |
| GLYCERIN | 3.00 | 3.00 | 3.00 | 3.00 | 3.00 |
| CALCIUM CHLORIDE 2H2O | | | 0.33 | 0.66 | 0.33 |
| KONJAC GUM | 1.50 | 2.00 | 1.00 | 0.50 | 1.50 |
| TARA GUM | 0.50 | | 0.50 | 0.50 | |
| Hydagen® 558 P * | | | 0.50 | 1.00 | 0.50 |
| CITRIC ACID 50% | 0.15 | 0.19 | 0.09 | 0.16 | 0.13 |
| | | | | | |
| Mixture no. | 213 | 214 | 215 | -216 | 217 |
| Ingredients | Mass Fraction [%] | Mass Fraction [%] | Mass Fraction [%] | Mass Fraction [%] | Mass Fraction [%] |
| WATER DEMIN | 94.17 | 93.52 | 93.70 | 94.04 | 93.04 |
| SODIUM BENZOATE | 0.50 | 0.50 | 0.50 | 0.50 | 0.50 |
| GLYCERIN | 3.00 | 3.00 | 3.00 | 3.00 | 3.00 |
| CALCIUM CHLORIDE 2H2O | 0.22 | 0.88 | 0.66 | 0.33 | 1.33 |
| KONJAC GUM | 0.33 | 0.33 | 1.00 | | |
| TARA GUM | 1.33 | 0.33 | | 1.50 | |
| Hydagen® 558 P * | 0.33 | 1.33 | 1.00 | 0.50 | 2.00 |
| CITRIC ACID 50% | 0.12 | 0.11 | 0.14 | 0.13 | 0.13 |

| Mixture no. | 208 | 209 | 210 | 211 | 212 |
|---|---|---|---|---|---|
| Ingredients | Mass Fraction [%] | Mass Fraction [%] | Mass Fraction [%] | Mass Fraction [%] | Mass Fraction [%] |
| WATER DEMIN | 93.70 | 93.92 | 94.34 | 94.13 | 93.35 |
| SODIUM BENZOATE | 0.50 | 0.50 | 0.50 | 0.50 | 0.50 |
| GLYCERIN | 3.00 | 3.00 | 3.00 | 3.00 | 3.00 |
| CALCIUM CHLORIDE 2H2O | 0.66 | 0.44 | | 0.22 | 1.00 |
| KONJAC GUM | | 0.66 | 1.00 | 1.33 | 0.50 |
| TARA GUM | 1.00 | 0.66 | 1.00 | 0.33 | |
| Hydagen® 558 P * | 1.00 | 0.66 | | 0.33 | 1.50 |
| CITRIC ACID 50% | 0.14 | 0.16 | 0.16 | 0.16 | 0.15 |
| | | | | | |
| Mixture no. | 218 | 219 | 220 | 221 | |
| Ingredients | Mass Fraction [%] | Mass Fraction [%] | Mass Fraction [%] | Mass Fraction [%] | |
| WATER DEMIN | 93.37 | 94.31 | 94.37 | 93.97 | |
| SODIUM BENZOATE | 0.50 | 0.50 | 0.50 | 0.50 | |
| GLYCERIN | 3.00 | 3.00 | 3.00 | 3.00 | |
| CALCIUM CHLORIDE 2H2O | 1.00 | | | 0.33 | |
| KONJAC GUM | | 0.50 | | 0.50 | |
| TARA GUM | 0.50 | 1.50 | 2.00 | 1.00 | |
| Hydagen® 558 P * | 1.50 | | | 0.50 | |
| CITRIC ACID 50% | 0.13 | 0.19 | 0.13 | 0.20 | |
| * Algin | | | | | |

The four properties described before have been determined at the 19 points in the system described in the previous tables. As described before, mathematical models have been developed based on these 19 points for the four properties.

As described before, the optimal areas in the ternary plots (diagrams) representing the ternary system examined, are defined by the overlap of the minimal requirements in these diagrams of all four properties.

| 203-221 | Texture | CR 24 h | BS | Number of Flakes | Particle size | Area fraction |
|---|---|---|---|---|---|---|
| Verdessence® Glucomannan Verdessence® Tara Verdessence® Alginate (total polymer content: 2 %) | | | | | | |
| widest (2) | <=1.5 | >95 | >300 | <=1200 | <=124 | <=0.26 |
| preferred (3) | <=1.25 | >95 | >400 | <=900 | <=116 | <=0.2 |
| More preferred (4) | =1 | >95 | >=400 | <=900 | <=116 | <=0.2 |

It was possible to achieve texture-ratings which were better than the texture-rating achieved with xanthan gum alone (set to 3.0 as a reference), while, at the same time, high curl retention (CR), high bending stiffness (BS) and low flaking could be achieved. Texture-ratings of 1.5 or lower could be achieved.

## Drawings

The present document comprises Fig. 2 to 4 and 14 to 16.

The figures of the present document are ternary plots showing the compositions that have the properties given in the table at the end of the experimental section. The widest range defines compositions according to the present invention, Narrower ranges represent preferred and more preferred compositions. The corners of the ternary plots represent 100 % by weight konjac gum (K), tara gum (T) and algin (A). The edges of the triangles of the ternary plots are linearly scaled and they represent binary mixtures. The hatched areas represent those compositions that have the properties given in the table at the end of the experimental section. Ternary plots are explained in detail in the English version of www.wikipedia.org under "ternary plot".

In Fig. 2 each point of the hatched area represents a composition of the system Tar, Alg, Glu which has the properties given as "widest" in the corresponding table at the end of the experimental section. This hatched area represents the preferred Tar-Alg-Glu-compositions according to the present invention.

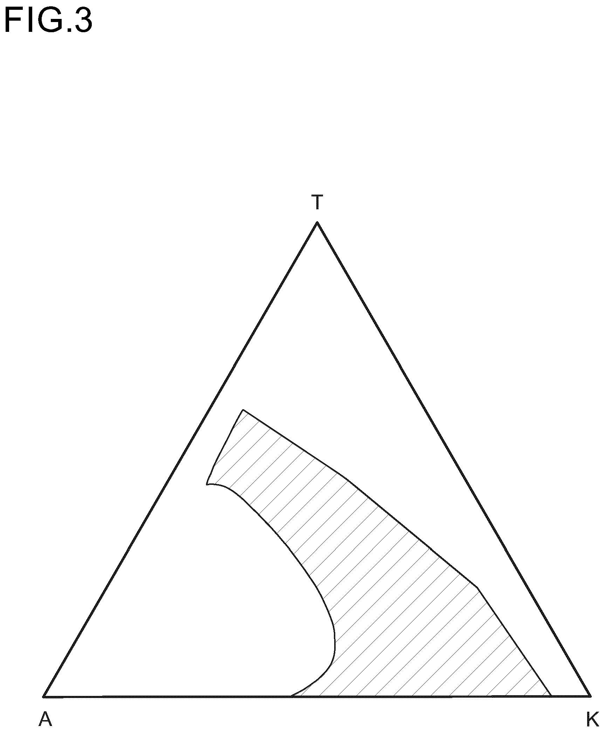

In Fig. 3 each point of the hatched area represents a composition of the system Tar, Alg, Glu which has the properties given as "preferred" in the corresponding table at the end of the experimental section. This hatched area represents the more preferred Tar-Alg-Glu-compositions according to the present invention.

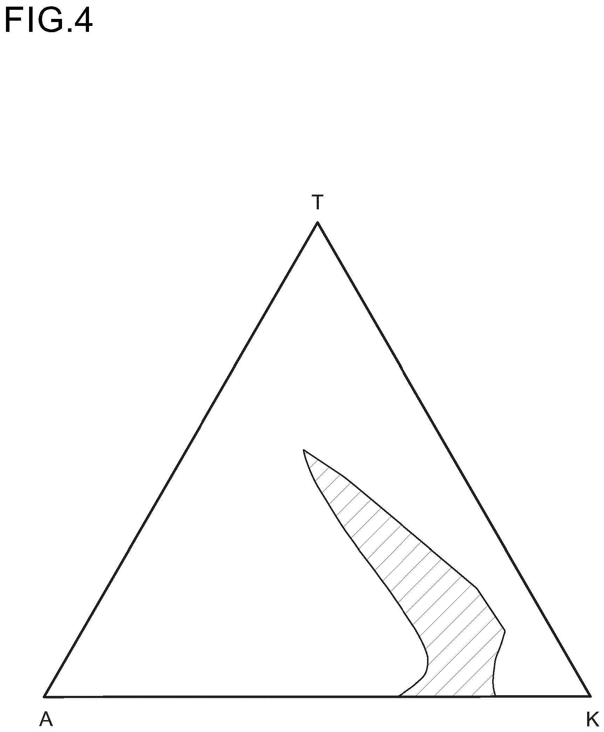

In Fig. 4 each point of the hatched area represents a composition of the system Tar, Alg, Glu which has the properties given as "more preferred" in the corresponding table at the end of the experimental section. This hatched area represents the most preferred Tar-Alg-Glu-compositions according to the present invention.

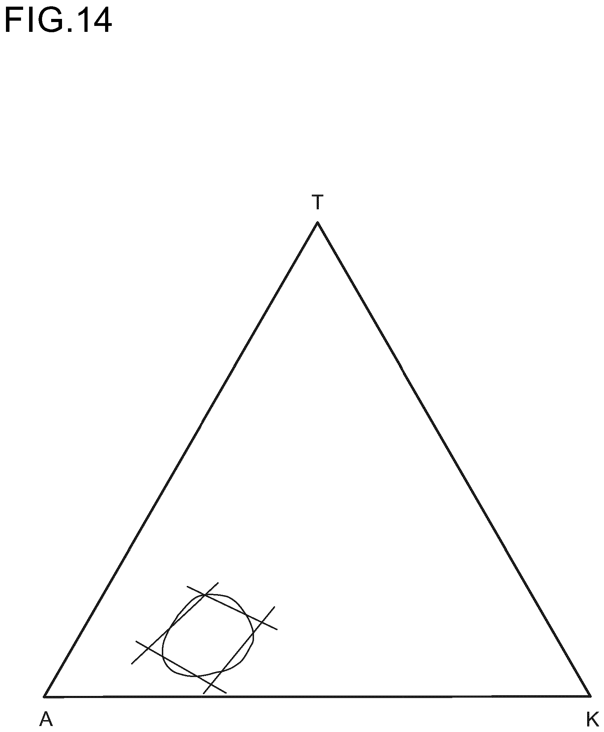

Fig. 14 to 16 correspond to Fig. 2 to 4. In Fig. 2 to 4 the hatched areas are bounded by curves. In Fig. 14 to 16 the hatched areas are approximated by polygons bounded by straight lines.

The polygons in Fig. 14 to 16 are defined by the positions of their corners in the corresponding ternary plot. The following paragraphs are listings of these corner-positions. The numbers represent the ratio of the polymer amounts.

Fig. 14: Tar, Alg, Glu (203-221) widest; Tar, Alg, Glu represented by x, y z:

All mixtures in the diagram area containing at least 2 of the 3 polymers in amounts represented by points outside of the following polygon.
Polygon 1: $X_5=0.21$, $y_5=0.60$, $z_5=0.19$ to $x_6=0.16$, $y_6=0.52$, $z_6=0.32$ to $x_7=0.03$, $y_7=0.68$, $z_7=0.29$, to $x_8=0.10$, $y_8=0.76$, $z_8=0.14$ and back to $x_5$, $y_5$, $z_5$.

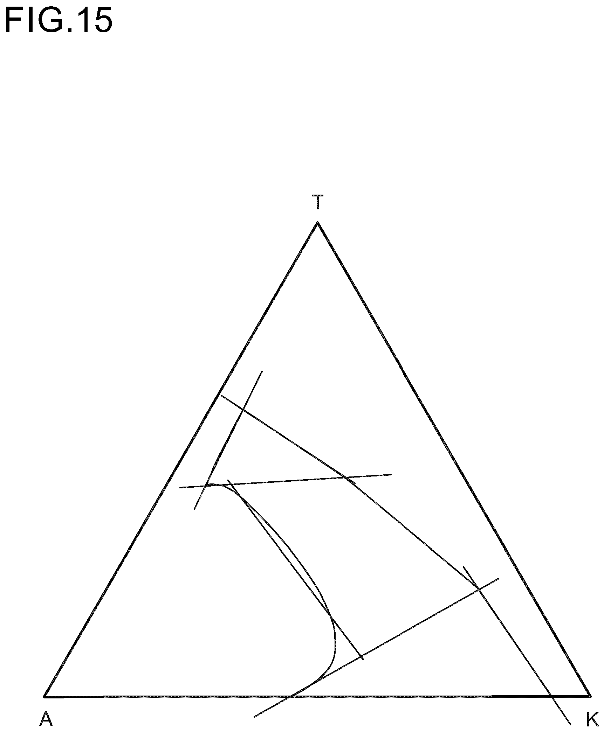

Fig. 15: Tar, Alg, Glu (203-221) preferred; Tar, Alg, Glu represented by x, y z:
All mixtures within the following polygons:

Polygon1: $x_1=0.45$, $y_1=0.48$, $z_1=0.07$ to $x_2=0.61$, $y_2=0.33$, $z_2=0.06$ to $x_3=0.46$, $y_3=0.22$, $z_3=0.32$ and back to $x_1$, $y_1$, $z_1$.
Polygon 2: $x_4=0.46$, $y_4=0.23$, $z_4=0.31$ to $x_8=0.23$, $y_8=0.09$, $z_8=0.68$ to $x_6=0.09$, $y_6=0.38$, $z_6=0.53$ to $x_7=0.45$, $y_7=0.43$, $z_7=0.12$ and back to $x_4$, $y_4$, $z_4$.
Polygon 3: $x_8=0.23$, $y_8=0.09$, $z_8=0.68$ to $x_9= 0$, $y_9=0.07$, $z_9=0.93$ to $x_{10}=0$, $y_{10}=0.55$, $z_{10}=0.45$ and back to $x_8$, $y_8$ and $z_8$.

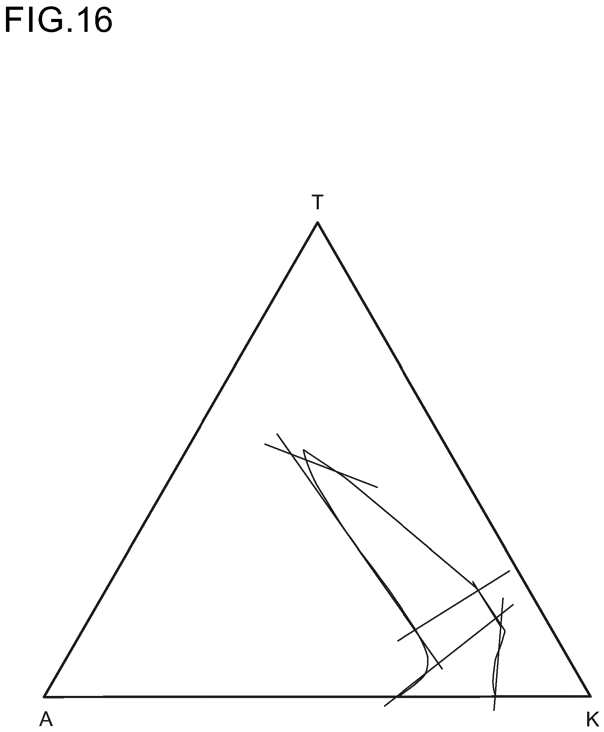

Fig. 16: Tar, Alg, Glu (203-221) more preferred; Tar, Alg, Glu represented by x, y z:
All mixtures within the following polygons:

Polygon 1: $x_1=0.14$, $y_1=0.25$, $z_1=0.61$ to $x_2=0.51$, $y_2=0.29$, $z_2=0.20$ to $x_3=0.47$, $y_3=0.23$, $z_3=0.30$ to $x_5=0.22$, $y_5=0.09$, $z_5=0.68$ and back to $x_1$, $y_1$, $z_1$.
Polygon 2: $x_5=0.22$, $y_5=0.09$, $z_5=0.68$ to $x_6=0.17$, $y_6=0.09$, $z_6=0.74$, to $x_7=0.07$, $y_7=0.24$, $z_7=0.69$ to $x_1=0.14$, $y_1=0.25$, $z_1=0.61$ and back to $x_5$, $y_5$, $z_5$.
Polygon 3: $x_8=0.18$, $y_8=0.07$, $z_8=0.75$ to $x_9=0$, $y_9=0.17$, $z_9=0.83$ to $x_{10}=0$, $y_{10}=0.35$, $z_{10}=0.65$ and back to $x_8$, $y_8$, $z_8$.

## Claims

1. A composition suitable for a cosmetic application comprising

   at least two of the components konjac gum, tara gum and algin,
   and furthermore comprising water,
   and furthermore comprising at least one further cosmetically acceptable ingredient different from the components comprised in the composition and different from water,
wherein the relative amount of each component present is not higher than 99 % by weight of the sum of the amounts of all components present,
and wherein, the weight ratio of these components is within the hatched area of the ternary diagram shown in Fig. 2, preferably within the hatched area of the ternary diagram shown in Fig. 3, more preferably within the hatched area of the ternary diagram shown in Fig. 4.

2. A raw material composition

consisting of at least two of the components konjac gum, tara gum and algin,
wherein the weight ratio of these components is within the hatched area of the ternary diagram shown in Fig. 2, preferably within the hatched area of the ternary diagram shown in Fig. 3, more preferably within the hatched area of the ternary diagram shown in Fig. 4,
and wherein the relative amount of each component present is not higher than 99 % by weight of the sum of the amount of all components present.

3. The composition according to claim 1 comprising

0.2 to 5.0 % by weight, preferably 0.5 to 4.0 % by weight, more preferably 1.5 to 2.0 % by weight of the total of all components present as defined in claim 1, and
0.1 to 40.0 % by weight, preferably 0.5 to 30 % by weight, more preferably 1 to 20 % by weight of the at least one further cosmetically acceptable ingredient,
wherein the balance to 100 % by weight is water.

4. The composition according to claim 1 or 3, wherein the composition is a composition suitable for styling hair.

5. The composition according to claim 1 or 3 to 4, wherein the composition is a skin care composition.

6. The composition according to claim 1 or 3 to 5, wherein the at least one further cosmetic ingredient is selected from the group consisting of preservatives, humectants, neutralizing agents, calcium salts, emollients, emulsifiers, fragrances and actives, plasticizers, glycerin, sodium benzoate, citric acid, calcium chloride, conditioning agents and combinations thereof.

7. The use of the composition according to any of claims 1 or 3 to 4 or 6 for styling hair.

8. The use of the composition according to any of claims 1, 3, 5 or 6 for skin care.

9. A process for styling hair comprising contacting the hair to the composition according to any of claims 1 or 3 to 4 or 6.

10. A process for achieving caring-effects to skin comprising contacting the skin with the composition according to any of claims 1, 3, 5 or 6.

# FIG.2

# FIG.3

FIG.4

# FIG.14

# FIG.15

# FIG.16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- International Cosmetic Ingredient Dictionary and Handbook. Personal Care Products Council